# EUROPÄISCHE PATENTSCHRIFT

(11) **EP 2 052 383 B1**
(45) Veröffentlichungstag und Bekanntmachung des Hinweises auf die Patenterteilung: **25.04.2012**
(21) Anmeldenummer: 07801683.9
(22) Anmeldetag: 16.08.2007
(51) Int. Cl.: G10D 3/10

(54) **VERWENDUNG VON KÜNSTLICH HERGESTELLTER SPINNENSEIDE**
USE OF SYNTHETIC SPIDER SILK
UTILISATION DE SOIE D'ARAIGNÉE SYNTHÉTIQUE

(30) Priorität: 16.08.2006 DE 102006038445
(43) Veröffentlichungstag der Anmeldung: 29.04.2009
(73) Patentinhaber: Gustav Pirazzi&Comp. Kg, 63069 Offenbach (DE)
(72) Erfinder: MÜLLER-ZIERACH, Eva, 63150 Heusenstamm (DE); MÜLLER-ZIERACH, Volker, 63150 Heusenstamm (DE)
(74) Vertreter: Tergau & Walkenhorst
(86) Internationale Anmeldenummer: PCT/EP2007/007224
(87) Internationale Veröffentlichungsnummer: WO 2008/019843

(56) Entgegenhaltungen:
- US-A- 2 039 262
- US-A- 2 130 948
- US-A1- 2005 161 058
- HINMAN M B ET AL: "Synthetic spider silk: a modular fiber" TRENDS IN BIOTECHNOLOGY, ELSEVIER, AMSTERDAM, NL, Bd. 18, Nr. 9, 1. September 2000 (2000-09-01), Seiten 374-379, XP004214264 ISSN: 0167-7799

## Beschreibung

Die Erfindung bezieht sich auf die Verwendung von künstlich hergestellter Spinnenseide, insbesondere als Faden gefertigt.

Im Rahmen zurückliegender Entwicklungen wurde die künstliche Herstellung von Fadenmaterial vervollkommnet, das in seinen Eigenschaften dem Naturprodukt Spinnenseide weitgehend gleicht. Eine solche künstlich hergestellte Spinnenseide und das Verfahren zu deren künstlicher Synthese sind beispielsweise aus den Druckschriften EP 1 287 139 A, EP 1 413 585 A2, EP 1 609 801 A1, WO 2006/002853 A1 und WO 2006/008163 A2 bekannt. Diese bekannte künstlich hergestellte Spinnenseide ist zum Einsatz als Faden oder Material im medizinischen Sektor, zur Netz- oder Seilherstellung, zur Textil-, Leder- oder Papierherstellung oder auch im Oberflächenschutz vorgesehen. Die künstlich hergestellte Spinnenseide weist dabei sowohl hinsichtlich der erreichbaren Festigkeitswerte als auch hinsichtlich der erreichbaren Dehnbarkeiten besonders günstige Eigenschaften auf. Das Dokument US-A-2 039262 offenbart die Verwendung von Spinnenseide als Bestandteil einer Seite für ein Musikinstrument

Der Erfindung liegt nunmehr die Aufgabe zugrunde, eine besonders günstige Verwendung für derartige künstlich hergestellte Spinnenseide anzugeben, bei der insbesondere die hohe Beanspruchbarkeit und die guten physikalischen Eigenschaften dieser Spinnenseide besonders wirkungsvoll genutzt werden können.

Diese Aufgabe wird erfindungsgemäß gelöst, indem künstlich hergestellte Spinnenseide als Bestandteil einer Saite in einem Musikinstrument gemäß Anspruch 1 verwendet wird.

Saiten für Musikinstrumente werden zwar üblicherweise aus Polymerkunststoffen, Tierdärmen, Metalldrähten, Pflanzenfasern und/oder Tierhaaren hergestellt und in der Regel in Zupf- und Streichinstrumenten verwendet. Dadurch, dass die Saiten am Instrument unter einer gewissen Spannung angebracht sind und bei Gebrauch angeschlagen, gezupft oder gestrichen werden, kommt es jedoch zu einer hohen Abnutzung der Musiksaite. Das Nachspannen der Musiksaiten, insbesondere zum Stimmen des Musikinstruments mittels Spannung der Saite, und der Abnutzungsgrad führen zu Rissbildungen innerhalb der Saite und somit zum Bruch der ganzen Saite. Hinzu kommen noch Alterungserscheinungen des verwendeten Saitenmaterials. Eine relativ häufige Saitenauswechslung, gerade bei Orchesterinstrumenten, ist somit nicht zu vermeiden, um die spezifischen musikalischen Eigenschaften des Instruments, vor allem der Ton- und Klangqualität, und der Saite nicht zu beeinträchtigen.

Insbesondere im Hinblick auf die üblicherweise hohe Beanspruchung der Saiten und das dadurch bedingte relativ häufige Auswechseln von defekten Saiten sollte somit ein Saitenmaterial verwendet werden, das in seinen Materialeigenschaften besonders langlebig und belastbar ist. Zudem sollten auch die spezifischen musikalischen Anforderungen von Saiten erfüllt werden. Ein solches Material sollte einerseits durch die spezielle molekulare Anordnung sehr dehnbar und extrem belastbar sein und hohe Zugfestigkeit aufweisen, andererseits aber noch elastisch gemäß der musikalischen Spezifikationen sein. Vorteilhaft ist ein gutes Recyclingverhalten des Saitenmaterials. Wie sich überraschender Weise herausgestellt hat, können diese Materialeigenschaften und physikalischen Eigenschaften durch künstlich hergestellte Spinnenseide besonders weitgehend erreicht werden. Zudem weist künstlich hergestellte Spinnenseide als Bestandteil einer Saite noch einen Gewichtsvorteil gegenüber herkömmlichen Saitenmaterialien auf.

Im Hinblick auf die vorgesehene Verwendung ist es zweckmäßig, wenn die künstlich hergestellte Spinnenseide, als Bestandteil einer Saite gefertigt, durch geeignete Parameterwahl, beispielsweise hinsichtlich Dimensionierung oder Zusammensetzung, an die spezifischen musikalischen, tonartlichen oder klangtechnischen Anforderungen, sowie Dehnungs- und Zugfestigkeit, die an eine Musiksaite für Musikinstrumente gestellt werden, angepasst ist. Dadurch sind insbesondere ein guter musikalischer Ton, eine einfache Handhabung beim Spielen und zudem eine besonders lange Lebensdauer erreichbar.

Hochwertige Musiksaiten können insbesondere aus einem von einer Umspinnung umgebenen Kern aufgebaut sein. Die künstlich hergestellte Spinnenseide kann dabei für die Umspinnung verwendet werden. Die künstliche Spinnenseide hat in Festigkeits- und Dehnungswerten allerdings sogar bessere Eigenschaften als z.B. Kevlar (Aramidfaser) und ist somit besser spielbar. Besonders vorteilhaft zur Nutzung der günstigen Eigenschaften der Spinnenseide wird diese daher für den Kern derartig aufgebauter Musiksaiten verwendet.

Der Kern der Musiksaite ist dabei vorzugsweise aus einer Feinfadenstruktur gebildet, die als Multifilament vorliegt. Dabei sind vorzugsweise die Feinfäden aus der künstlich hergestellten Spinnenseide gefertigt. Zur Bildung des Multifilaments ist dabei eine Vielzahl dünner Einzelfeinfäden zu einer "Saite" verdrillt. Diese kann noch mit einem widerstandsfähigen Material ein- oder mehrlagig umsponnen sein. Vorteile sind hierbei höhere Elastizität und Spielbarkeit. Das Multifilament kann dabei alternativ auch in einer Struktur vorliegen, bei der die Feinfadenstruktur parallel und nicht verdrillt im Kern angeordnet ist.

Saiten für Musikinstrumente werden in ihrem technischen Saitenaufbau unterschieden. Monofilamentsaiten, wie z.B. einige Gitarrensaitentypen, haben einen dünneren Durchmesser und werden vorzugsweise als Saite zum Erzeugen von hohen Tönen verwendet. Die guten Eigenschaften der künstlich hergestellten Spinnenseide hinsichtlich der erreichbaren Festigkeitswerte und hinsichtlich der erreichbaren Dehnungswerte sind bei Monofilamentsaiten, mit ihrem dünneren Durchmesser und ihrem einstrangigen Saitenaufbau, von Vorteil.

Bei Saiten für Musikinstrumente, die einen Kern aufweisen, kann dieser auch vorteilhafterweise Bestandteile von künstlich hergestellter Spinnenseide aufweisen. Ist der Kern der Saite als hochwertiges Multifilament gefertigt wie z.B. einige Violinensaitentypen, so können die einzelnen Fäden, die das Multifilament bilden, vorzugsweise aus künstlich hergestellter Spinnenseide bestehen. Die insbesondere daraus resultierende Gewichtsersparnis und zudem die guten physikalischen Eigenschaften der künstlich hergestellten Spinnenseide sind bei Saiten, deren Kern ein Multifilament aufweist, von Vorteil.

Vorteilhafterweise ist die Verwendung von mindestens einer Saite, hergestellt aus künstlicher Spinnenseide, bei Musikinstrumenten, die der Streich- oder Zupfinstrumentenfamilie zugeordnet sind, vorgesehen.

Ein Musikinstrument, insbesondere ein Streichinstrument oder ein Zupfinstrument, mit einer Anzahl von Musiksaiten weist erfindungsgemäß zumindest eine oder einige Musiksaiten auf, die unter Verwendung künstlich hergestellter Spinnenseide hergestellt sind und/oder deren Bestandteil künstlich hergestellte Spinnenseide ist. Diese sind dabei vorteilhafterweise durch geeignete Parameterwahl, insbesondere hinsichtlich Dimensionierung und/oder Zusammensetzung, im Hinblick auf ihre klanglichen oder tonartlichen Eigenschaften an die vorgesehene Verwendung für Musiksaiten angepasst.

Die mit der Erfindung erzielten Vorteile bestehen insbesondere darin, dass die Verwendung von künstlich hergestellter Spinnenseide als Bestandteil einer Musiksaite einer hohen Belastung und Beanspruchung sowie Langlebigkeit gerecht wird. Damit können die günstigen Eigenschaften der künstlich hergestellten Spinnenseide, insbesondere die vergleichsweise hohe Festigkeit bei gleichzeitig besonders hoher Dehnbarkeit, besonders günstig genutzt werden. Desweiteren wird bei einer solchen Verwendung eine Gewichtseinsparung sowie gute physikalische Eigenschaften der Musiksaite erreicht.

Ein Ausführungsbeispiel der Erfindung wird anhand einer Zeichnung näher erläutert. Darin zeigen:
- Fig. 1: eine schematische Ansicht einer klassischen Violine,
- Fig. 2: eine Musiksaite für eine Violine mit angedeutetem Steg, Obersattel und Wirbel, und
- Fig. 3: die Musiksaite aus Fig. 2 im Querschnitt.

Gleiche Teile sind in allen Figuren mit denselben Bezugszeichen versehen.

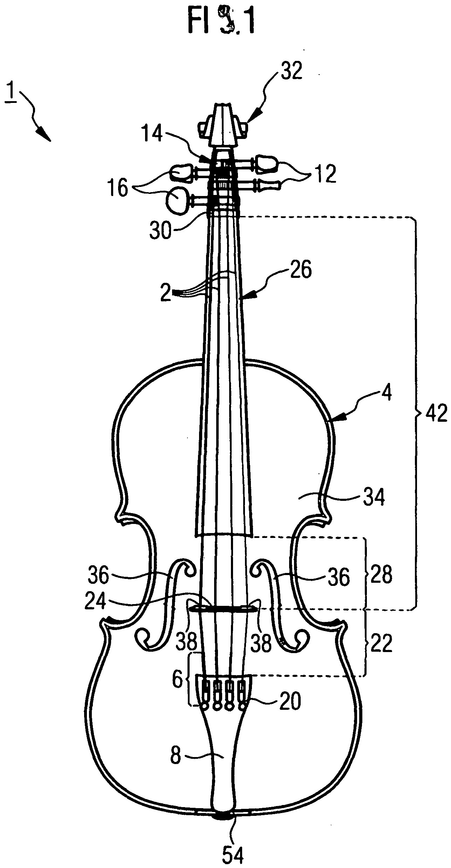

Eine klassische Violine 1 gemäß Fig. 1 umfasst vier Saiten 2, die über einen Resonanzkörper 4 gespannt sind. An einem ersten Ende, dem Saitenhalterende 6, sind die Saiten 2 an einem Saitenhalter 8 befestigt. An ihrem zweiten Ende, dem Wirbelende 10, sind sie hingegen auf Wirbeln 12 einer Stimmmechanik, dem sog. Wirbelkasten 14, befestigt. Dazu können die Wirbel 12 ein Loch aufweisen, durch welches das Wirbelende 10 der Saite 2 hindurchgesteckt ist. Das Wirbelende 10 ist dabei in seinem anschließenden Bereich mehrfach um den Wirbel 12 herumgelegt. Die Wirbel 12 sind über ihre Wirbelgriffplatten 16 verdrehbar, wodurch die Saitenspannung und damit der Ton der Saite 2 veränderbar ist.

Am Saitenhalterende 6 ist die Saite 2 mit einem Endkopf 18 versehen, der im Saitenhalter 8 befestigt ist. Zudem ist am Seitenhalter 8 ein Feinstimmer 20 befestigt, mit Hilfe dessen die Spannung jeder Saite 2 feinjustiert wird. Der Feinstimmer 20 dient dem Musiker den Ton der Saite 2 noch genauer einzustellen als dies über die Wirbel 12 möglich ist.

Der Aufspannabschnitt 22 der Saite 2 befindet sich zwischen Saitenhalter 8 und Steg 24 und ist ab dem Endkopf 18 im Bereich des Saitenhalterendes 6 zum Teil mit farbigem Garn angesponnen. Das farbige Garn hat den Zweck, die Saitenqualität anzuzeigen. Ebenfalls ist das Wirbelende 10 der Saite 2, das um den Wirbel 12 herumgeführt ist, mit farbigem Garn eingesponnen, das dem Musiker den genauen Tonbereich der Saite anzeigt.

Der Bereich zwischen Steg 24 und Griffbrett 26 ist der Spielbereich 28, in dem der Musiker die Saiten 2 zupft oder mit dem Bogen streicht. Zum Spielen der Violine greift der Musiker die verschiedenen Akkorde auf den Saiten 2 entlang des Griffbrett 26 und streicht die Saiten 2 mit dem Bogen im Spielbereich 28.

Am anderen Ende des Griffbretts 26 ist ein Obersattel 30 angebracht, über den die Saiten 2 zu den Wirbeln 12 im Wirbelkasten 14 geführt sind. Den Abschluss des Wirbelkastens 14, und somit auch der Violine, bildet die Schnecke 32.

In der Decke 34 des Resonanzkörpers 4 sind die beiden f-Löcher 36 der Violine seitlich in Höhe des Stegs 24 eingebracht. Die f-Löcher 36 dienen zum ungehinderten Bewegen der eingeschlossenen Luft im Resonanzkörper, und des weiteren wird die Schwingungsfähigkeit der Decke 34 im akustischen Zentrum um die Stegfüße 38 wesentlich erhöht.

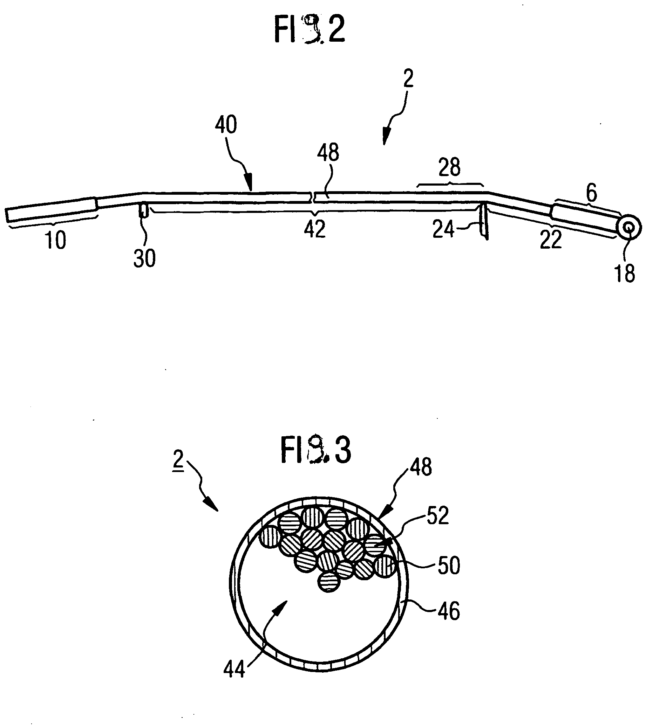

Der äußerliche Aufbau der Saite 2 wird anhand Fig. 2 erläutert. In dieser sind der Endknopf 18 mit zum Teil in farbigem Garn eingespanntem Aufspannabschnitt 22, der das Saitenhalterende 6 bildet, und der meist polierte Saitenkörper 40 mit anschließendem Wirbelende 10 der Saite, ebenfalls in farbigem Garn eingesponnen, zu sehen. In perspektivischer Ansicht sind Steg 24 und Obersattel 30 angedeutet. Der meist polierte Saitenkörper 40 ist der tonerzeugende Abschnitt 42, der den Spielbereich 28 und den Bereich der Saite 2 über dem Griffbrett 26 bis zum Obersattel 30 bildet.

Den inneren Aufbau der Saiten 2 zeigt die Darstellung im Querschnitt nach Fig. 3. Die Saite 2 ist mit einem Kern 44 und einer Umspinnung 46 ausgeführt. Die Umspinnung 46, z.B. aus Metall oder Kunststoff, ist an ihrem Außenmantel 48, je nach verwendetem Material, meist poliert. Mit bis zu zehn Umspinnungslagen ist die Umspinnung 46 fest um den Kern 44 gesponnen.

Der Kern 44 umfasst eine Vielzahl von im wesentlichen parallel geführten Feinstfäden 50, die aus künstlich hergestellter Spinnenseide bestehen, und bilden das Multifilament 52 der Saite 2.

Die Feinstfäden 50 im Multifilament 52 könnten miteinander verseilt oder verknüpft sein. Im Ausführungsbeispiel sind sie aber in gerader Richtung parallel geführt oder etwas einseitig verdrillt, wobei die einseitige Verdrillung bis ca. 100 Umdrehungen pro Meter Saite 2 betragen kann.

Hinsichtlich der Herstellung und der Verarbeitung der Feinstfäden 50 werden die guten physikalischen Eigenschaften, die erreichbaren Druck- und Zugbelastung, Elastizität und Reisfestigkeit, der künstlich hergestellten Sinnenseide genutzt. Ebenso bei der Verwendung von künstlich hergestellter Spinnenseide als Umspinnung 46 der Saite 2 kommen die erreichbaren guten physikalischen Eigenschaften von künstlich hergestellter Spinnenseide zur Anwendung.

Des weiteren ist die Saite 2, bestehend aus dem Multifilament 52 und der Umspinnung 46, die als ein Bestandteil künstlich hergestellte Spinnenseide aufweist, leichter und hat eine höhere Lebensdauer. Zudem ist eine solche Saite 2 durch ihre gute Dehnungs- und Zugfestigkeit besser spielbar und einfach in der Handhabung.

Die Violine 1 ist unter anderem für eine besonders lange Lebensdauer und eine hohe musikalische Qualität der verwendeten Saite 2 ausgelegt. Dazu sind einige oder alle Saiten 2 mit einem oder mehren Bestandteilen aus künstlich hergestellter Spinnenseide gefertigt, die durch geeignete Parameterwahl, beispielsweise Zusammensetzung und Fadendicke, spezifisch an die Anforderungen der jeweiligen Saite 2 angepasst sind.

Der Saitenhalter 8 ist in seinem Endbereich 54 über eine so genannte Anhängesaite unter Vorspannung an einem Halteknopf befestigt. Auch für diese Anhängesaite kann hinsichtlich hergestellte Spinnseide als Materialkomponente vorgesehen sein.

In den Figuren ist die Verwendung der Spinnenseide als Musiksaite am Beispiel einer Saite 2 in der Violine 1 und als Saite 2 mit Multifilament 52 dargestellt. Selbstverständlich umfasst die Erfindung auch die Verwendung von künstlich hergestellter Spinnenseide als Bestandteil einer Musiksaite in beliebigen anderen Saiteninstrumenten, insbesondere Streich- und Zupfinstrumenten.

### Bezugszeichenliste

- 1: Violine
- 2: Saite
- 4: Resonanzkörper
- 6: Saitenhalterende
- 8: Saitenhalter
- 10: Wirbelende
- 12: Wirbel
- 14: Wirbelkasten
- 16: Wirbelgriffplatte
- 18: Endkopf
- 20: Feinstimmer
- 22: Aufspannabschnitt
- 24: Steg
- 26: Griffbrett
- 28: Spielbereich
- 30: Obersattel
- 32: Schnecke
- 34: Decke
- 36: f-Loch
- 38: Stegfüße
- 40: Saitenkörper
- 42: tonerzeugender Abschnitt
- 44: Kern
- 46: Umspinnung
- 48: Außenmantel
- 50: Feinstfäden
- 52: Multifilament
- 54: Endbereich

## Patentansprüche

1. Verwendung von künstlich hergestellter Spinnenseide, insbesondere als Faden gefertigt, als Bestandteil einer Saite (2) für ein Musikinstrument.

2. Verwendung von künstlicher Spinnenseide nach Anspruch 1 im Kern (44) der Saite (2).

3. Verwendung von künstlicher Spinnenseide nach Anspruch 1 oder 2, bei der die Feinfadenstruktur aus künstlich hergestellter Spinnenseide im Kern (44) der Saite (2) als Multifilament (52) vorliegt.

4. Saite (2) für ein Musikinstrument, die als einen ihrer Bestandteile künstlich hergestellte Spinnenseide aufweist.

5. Saite (2) nach Anspruch 4, bei der die künstlich hergestellte Spinnenseide Bestandteil ihres Kerns (44) ist.

6. Saite (2) nach Anspruch 4 oder 5, bei der die künstlich hergestellte Spinnenseide als Multifilament (52) vorliegt.

7. Musikinstrument, insbesondere Streich- oder Zupfinstrument, mit einer Anzahl von Saiten (2) nach einem der Ansprüche 4 bis 6.

## Claims

1. Use of synthetic spider silk, in particular manufactured as a thread, as a component of a string (2) for a musical instrument.

2. Use of synthetic spider silk according to claim 1 in the core (44) of the string (2).

3. Use of synthetic spider silk according to claim 1 or 2, wherein the fine-thread structure made from synthetic spider silk in the core (44) of the string (2) is provided as a multifilament (52).

4. A string (2) for a musical instrument, including synthetic spider silk as one of its components.

5. The string (2) according to claim 4, wherein the synthetic spider silk is a component of its core (44).

6. The string (2) according to claim 4 or 5, wherein the synthetic spider silk is provided as a multifilament (52).

7. A musical instrument, in particular a bowed or a plucked instrument, having a number of strings (2) according to any of claims 4 to 6.

## Revendications

1. Utilisation de soie d'araignée synthétique, en particulier fabriquée comme un fil, en tant que composante d'une corde (2) pour un instrument de musique.

2. Utilisation de soie d'araignée synthétique selon la revendication 1 dans le noyau (44) de la corde (2).

3. Utilisation de soie d'araignée synthétique selon la revendication 1 ou 2, dans laquelle la structure de fil fin faite de soie d'araignée synthétique dans le noyau (44) de la corde (2) est prévue comme un multifilament (52).

4. Corde (2) pour un instrument de musique, incluant de la soie d'araignée synthétique comme une de ses componsantes.

5. Corde (2) selon la revendication 4, dans laquelle la soie d'araignée synthétique est une composante de son noyau (44).

6. Corde (2) selon la revendication 4 ou 5, dans laquelle la soie d'araignée synthétique est prévue comme un multifilament (52).

7. Instrument de musique, en particulier un instrument à cordes frottées ou un instrument à cordes pincées, ayant u nombre de cordes (2) selon l'une quelconque des revendications 4 à 6.
